# EUROPEAN PATENT APPLICATION

(11) **EP 0 550 754 A1**
(43) Date of publication of application: **14.07.1993**
(21) Application number: 92903381.9
(22) Date of filing: 24.07.1991
(51) Int. Cl.: C07C 7/20, C07C 15/44, C07C 15/46, C07D 213/06

(54) **POLYMERIZATION INHIBITOR OF VINYL-CONTAINING COMPOUNDS**

(71) Applicant: NIZHNEKAMSKOE PROIZVODSTVENNOE OBIEDENENIE " NIZHNEKAMSKNEFTEKHIM", Nizhnekamsk, 423550 (SU)
(72) Inventor: KURBATOV, Vladimir Anatolievich, Moscow, 112059 (SU); BOREIKO, Natalya Pavlovna, Nizhnekamsk, 423550 (SU); ZUEV, Valery Pavlovich, Nizhnekamsk, 423550 (SU); LIAKUMOVICH, Alexandr Grigorievich, Kazan, 420072 (SU)
(74) Representative: W.P. Thompson & Co.
(86) International application number: SU9100156
(87) International publication number: WO9302028

(57) **Abstract**

A polymerization inhibitor of vinyl-containing compounds consists of the product of interaction between 4-(dimethylamino)methyl-2,6-bis-(trebutyl)phenol and a non-organic acid or carbonic acid or an anhydride of an organic acid. The weight ratio between said components is 1-15:0, respectively. The process is carried out in the presence of a solvent at a temperature of up 120 °C.

## Description

### Technical Field

The invention relates to organic synthesis, particularly, to inhibitors of polymerization of vinyl-containing compounds.

### Prior Art

In the process of obtaining of vinyl-containing compounds, such as, for instance, styrene, divinylbenzene, methylvinylpyridine at the stage of their isolation and purification, their spontaneous polymerization takes place which leads to clogging of the process equipment with polymers thereby reducing heat transfer, output and causing out-of-plan stoppage of the production process.

To avoid undesirable polymerization of monomers use is made of inhibitors. Such inhibitors must reliably inhibit the polymerization of monomers within a wide temperature range. They must be readily soluble in hydrocarbon solvents, since otherwise, the process accessories will be clogged. Inhibitors with insufficient solubility are used in the form of suspension, which requires more complicated process equipment, particularly, special equipment intended for the process.

Moreover, the inhibitor must be inactive with respect to vinyl-containing compounds and must not cause undesirable side effects when salvaging production wastes of the vinyl-containing monomers (pollution of the environment, corrosion of the equipment).

The inhibitor should not be toxic, explosive and fire-hazardous.

Known in the art are inhibitors of polymerization of vinyl-containing compounds belonging to various classes of chemical compounds. The most widely-used among them are quinones, nitro- and nitrozocompounds, amines.

However, few of them have found practical application, particularly, sulfur, mixtures of n-quinonedioxime with hydroquinone or with n-nitrophenol, dinitrocompounds.

Thus, for example, known in the art is the use of sulfur as an inhibitor for styrene polymerization (US, A, 4050993). Sulfur is sufficiently effective for inhibiting polymerization of monomers with the dosage of from 3 to 5 kg/t of monomer. However, large losses of styrene are inevitable due to formation of its sulfur-containing compounds, which, moreover, cause corrosion of the process equipment. Besides, salvaging of still bottoms which remain from the process of styrene rectification leads to pollution by sulfur oxides due to their combustion.

Inhibitors containing n-quinonedioxime, for instance, a mixture of the latter with hydroquinone (SU, A, 257496) or n-nitrophenol (SU, A, 441263) reliably inhibit spontaneous polymerization of monomers within a wide range of temperatures. However, they feature but a low solubility in aromatic hydrocarbons and this causes clogging of conveying pipelines, disks and reboilers of columns by the inhibitor. Use of suspensions - quinonedioxime requires special power consuming equipment. In addition, n-quinonedioxime is combustible and its air-dust mixtures are explosive.

Dinitrophenols (US, A, 4033829, US, A, 3959395)also meet the main requirements of the inhibitor characteristics, that is, they are effective within a wide range of temperatures. However, these compounds are toxic and fire-hazardous as those mentioned above.

Thus, up to now, there is no known inhibitor of polymerization of vinyl-containing compounds which meets all the demands described above.

### Disclosure of the Invention

It is an object of the invention to provide an inhibitor of polymerization of vinyl-containing compounds that would be operable within a wide temperature range, readily dissolvable in hydrocarbon solvents, inactive to a monomer, not toxic, fire - and explosion-safe.

This object is attained by the provision of an inhibitor of polymerization of vinyl-containing compounds which, according to the invention, is essentially a product of interreaction of 4-(dimethylamino)methyl-2-,6-bis(tertbutyl) phenol with a compound selected from an inorganic acid, carboxylic acid and anhydride of an organic acid, with the weight ratio of said components being from 1 -15: 3, respectively, in an organic solvent at a temperature not exceeding 120°C.

Used as an inorganic acid may be, for instance, hydrochloric acid, phosphoric acid, bromohydric acid.

Used as a carboxylic acid may be adipic, maleic, palmitic, acetic, phthalic, synthetic fatty acids and, as anhydrides of organic acids, acetic, maleic, phthalic anhydrides.

As an organic solvent use may be made of hydrocarbon solvent, such as for instance, benzene, toluene, ethylbenzene, xylene, acetophenone, methylphenilcarbinol and others, as well as vinyl-containing compounds, for instance, styrene, methyl-vinylpyridine.

The choice of the solvent depends on the terms of storage of the inibitor. In case the inhibitor has to be stored for a prolonged time period, preferably hydrocarbon solvent is used. If the inhibitor is used right after it is produced, more advantageously vinyl-containing compounds are used as an organic solvent.

Said weight ratio of the components, namely, 1-15:3 is the optimum.

It ensures sufficient operability of the inhibitor within a wide range of its concentrations. With the weight ratio of the components less than 1:3, the inhibitor content is reduced,which leads, respectively, to the reduction of its effectiveness, whereas with the enhanced ratio more than 15:3 (5:1), the utilization rate of the inhibitor increases without further increase of its effect.

The choice of the temperature range at the inhibitor preparation stage depends on its concentration: more concentrated solutions require higher temperature.

The proposed inhibitor of polymerization of vinyl-containing compounds is effective within a wide temperature range (up to 120°C), is readily soluble in hydrocarbon solvents and vinyl-containing compounds,which allows its use in any concentrations.

The proposed inhibitor of polymerization of vinyl-containing compounds is inactive to monomers, and is fire-and explosion-safe product.

Original raw stock for obtaining the inhibitor are readily available and inexpensive products.

### Preferred Embodiment of the Invention

A method of producing the polymerization inhibitor of vinyl-containing compounds is simple in production processes and is carried out as follows.

A container provided with an agitator and a thermometer is charged with 4-(dimethylamino) methyl-2,6-bis (tertbutyl)phenol, the second component, which is an inorganic acid, or carboxylic acid, or an anhydride of the latter, and an organic solvent. The mixture thus obtained is stirred and, if necessary, heated to an adopted temperature under constant stirring until the components are completely dissolved, 4-(dimethylamino) methyl-2,6-bis(tertbutyl) phenol is obtained by Mannich reaction consisting in interreaction of 2,6-ditertbutylphenol, formaldehyde and dimethylamine.

For a bether understanding of the present invention given below are particular examples of its embodiment.

### Example 1

Charged into a three-neck flask provided with an agitator is 10 g of 4-(dimethylamino)methyl-2,6-bis (tertbutyl) phenol, 5 g of adipic acid and 85 g of toluene. The mixture is stirred at a temperature of 60°C for 30 minutes.

To test the inhibitor thus obtained for efficacy, charged into a three-neck flask provided with a reflux condenser, a thermometer and a capillary for feeding nitrogen, is 300 ml of styrene with the content of basic substance being 99.85 wt %, and 0.27g of the obtained inhibitor (which in terms of dry substance equals 0.015 wt%). The flask contents is heated in a stream of nitrogen to a temperature of 100°C under residual pressure of 215 mm Hg for 3 hours. Then the pressure in the flask is raised to the atmospheric and a sample is taken to carry out a test for the styrene polymer content by the nephelometric method. No polymer is present.

### Example 2

The inhibitor is obtained as described above in Example 1, with the sole exception that used as a solvent is xylene.

The inhibitor obtained is tested for efficacy also under the conditions of polymerization of styrene. The conditions of the test for efficacy of the inhibitor differ from those described in Example 1 by that the temperature of the process is 110°C, and the amount of the inhibitor is 0.81 g. In 3 hours the styrene polymer content equals 0.002 wt % and in 5 hours, 0.012 wt %.

### Example 3

Charged into a three-neck flask equipped with an agitator and a thermometer is 10 g of 4-(dimethylamino) methyl-2,6-bis (tertbutyl)phenol, 20 g of synthetic fatty acids (C₁₇-C₂₀) and 70 of ethylbenzene. The obtained mixture is heated under constant stirring and a temperature of 100°C for 2 hours.

The inhibitor is tested for efficacy under the conditions of styrene polymerization. To this end, charged into a three-neck flask equipped with a reflux condenser, a thermometer and a capillary for feeding nitrogen, is 300 ml of styrene and 1.35 g of the obtained inhibitor (in terms of dry substance, 0.075 wt %).

In the stream of nitrogen at a temperature of 115°C and under residual pressure of 215 mm Hg, styrene is kept for 5 hours. Then pressure in the flask is raised to the atmospheric one and a sample is taken for the test of polymer content using nephelometric method. No polymer is present. After 6 hours of heating its content equals 0.12 wt %.

### Example 4

Charged into a three-neck flask fitted with an agitator and a thermometer, is 1 g of 4-(dimethylamino) methyl-2,6-bis (tertubyl)phonol, 0,2 g of acetic acid and 98.8 g of styrene.

The mixture is stirred at a temperature of 20°C for 10 minutes.

The inhibitor thus obtained is tested for efficacy under the conditions of styrene polymerization as in Example 1, with the exception that the inhibitor is added in the amount of 2.7 g. After 6 hours of heating the styrene polymer content amounts to 0.004 wt %.

### Example 5

The inhibitor is prepared as in Example 1, with the exception that phthalic acid is used.

The obtained inhibitor is tested in the process of styrene polymerization as described in Example 1. In 6 hours of heating of styrene the polymer content in it amounts to 0.02 wt %.

### Example 6

The inhibitor is prepared as described in Example 1, with the exception that use is made of maleic anhydride, and benzene is used as an organic solvent.

The prepared inhibitor is tested for efficacy under the conditions of styrene polymerization, as described in Example 1. After 6 hours of heating of styrene, the polymer content in it equals 0.013 wt %,

### Example 7

The inhibitor is prepared as described in Example 1, with the exception that use is made of acetic anhydride and the process is carried out at a temperature of 18°C.

The obtained inhibitor is tested under the conditions of polymerization of styrene. After 6 hours of heating the content of polystyrene amounts to 0.006 wt%.

### Example 8

Charged into a round-bottom flask provided with an agitator and a thermometer is 5 g of 4-(dimethylamino) methyl-2,6-bis(tertbutyl)phenol, 5 g of palmitic acid, 90 g of ethylbenzene. The mixture is stirred at a temperature of 80°C for 1 hour.

The obtained inhibitor is tested for efficacy under the conditions of polymerization of styrene. To this end, charged into a three-neck flask provided with a reflux condenser, a thermometer and a capillary for feeding nitrogen, is 300 ml of styrene, 2.7 g of inhibitor. The flask contents is heated to 120°C in the nitrogen stream under residual pressure of 205 mm Hg for 6 hours. After this time period is over, the pressure in the flask is raised to atmospheric and the content of polymer in styrene is found using nephelometric method, which amounts to 0.4 wt %.

### Example 9

In an alchohol-washed, thoroughly nitrogen-blown, weighed ampoule, load 0.79 g of 4-(dimethylamino) methyl-2,6,-bis (tertbutyl) phenol, 0.22 g of hydrochloric acid and 2 g of methylvinylpyridine. The ampoule is shaken. The inhibitor thus obtained is tested for efficacy, for which purpose 8 g of methyl-vinylpyridine (freshly distilled product with basic substance content being 91 wt %) is added into the ampoule under the conditions of polymerization of methylvinylpyridine. The ampoule is sealed up and thermostatted at a temperature of 110°C for 15 hours. Then the ampoule is unsealed, its contents mixed with heptane and the content of the polymer found. If amounts to 0.35 wt %.

### Example 10

The inhibitor is prepared as described in Example 1. The obtained inhibitor is tested under the conditions of polymerization of divinylbenzene, contained in the mixture of the following composition, wt %.

| | |
|---|---|
| divinylbenzene | 55.314 |
| ethylbenzene | 0.013 |
| diethylbenzene | 2.590 |
| styrene | 1.140 |
| toluene | 0.029 |
| benzene | 0.011 |
| paraffin hydrocarbons | 0.019 |
| ethylstyrene | 40.884 |

Charged into a three-neck flask provided with a reflux condenser, a thermometer, and a capillary for feeding nitrogen is 300 ml of said mixture and 0.81 g of the inhibitor. The flask contents is heated in the nitrogen stream under residual pressure of 300 mm Hg and at a temperature of 120°C for 4.5 hours. Then the pressure is increased to atmospheric and a sample is taken to carry out the test for polymer content by gravimetric method. The content of the polymer amounts to 0.07 wt %

### Example 11

The inhibitor is prepared as described in Example 1 with the exception that the temperature of the process is 32°C, and used as an organic solvent is styrene.

The inhibitor is tested for efficacy under the conditions of polymerization of styrene.

After 3 hours of heating no polymer is present, and in 5 hours its content amounts to 0.07 wt %.

### Industrial Applicability

The proposed inhibitor of polymerization of vinyl-containing compounds is applicable to prevent undesirable spontaneous polymerization of such monomers as styrene, methylvinyl-pyridine, divinylbenzene, in the process of their isolation and purification.

## Claims

1. A polymerization inhibitor of vinyl-containing compounds, characterized in that it is essentially the product of interreaction of 4-(dimethylamino) methyl-2,6-bis (tertbutyl)phenol with a compound selected from an inorganic acid, carboxylic acid and anhydride of an organic acid, the weight ratio of said components being 1-15:3, respectively, in an organic solvent at a temperature not exceeding 120°C.
